# EUROPEAN PATENT APPLICATION

(11) **EP 4 527 919 A1**
(43) Date of publication of application: **26.03.2025**
(21) Application number: 23807891.9
(22) Date of filing: 16.05.2023
(51) Int. Cl.: C12N 1/20, A61K 35/74, A61P 25/20, C12R 1/01

(54) **PREVOTELLA HISTICOLA**

(30) Priority: 16.05.2022 KR 20220059762; 16.05.2022 KR 20220059763; 16.05.2022 KR 20220059764
(71) Applicant: Kookmin University Industry Academy Cooperation Foundation, Seongbuk-gu Seoul 02707 (KR); Industry-Academic Cooperation Foundation, Chosun University, Gwangju 61452 (KR)
(72) Inventor: BAIK, Inkyung, Seoul 05811 (KR); KOOK, Joong Ki, Gwangju 61664 (KR); PARK, Soon Nang, Gwangju 61978 (KR); LIM, Yun Kyong, Gwangju 61256 (KR)
(74) Representative: V.O.
(86) International application number: PCT/KR2023/006647
(87) International publication number: WO 2023/224372

(57) **Abstract**

The present disclosure relates to a *Prevotella histicola strain* useful for the prevention or treatment of sleep disorders. Since *Prevotella histicola* increases total sleep duration, non-REM (NREM) sleep duration and REM sleep duration and decreases sleep latency and wake time, it can be used to prepare a safe drug for the prevention and treatment of sleep disorders.

## Description

### [Technical Field]

The present disclosure relates to a *Prevotella histicola strain* useful for the prevention, amelioration or treatment of sleep disorders.

### [Background Art]

The U.S. market size for insomnia drugs is about 2 trillion won and has been growing annually at a rate of 20% or higher in recent years. The leading sleep disorder drug in the U.S. is Stilnox. Not only in the U.S., it also has a market share of about 50% in the Korean sleeping drug market. While sleeping drugs are generally effective for short-term use, long-term use of more than 4 weeks is associated with dependence and side effects, especially in older adults who have a higher prevalence of insomnia. Therefore, there is a growing interest in natural sleep aids as an alternative to sleeping drugs, and the related market is expanding. There are currently more than 10 natural sleep aids that are most commonly used in the United States and European countries, but there are very limited scientific reports on their effectiveness in treating insomnia. According to a consumer report in the United States, 64% of people who used sleeping drugs and 20% of people who used sleep aids experienced sleep enhancement, suggesting that sleep aids are very ineffective. In Korea, there are currently few natural sleep aids that are recognized by the Ministry of Food and Drug Safety as health functional foods for sleep promotion and improvement, and there is limited research on related functional ingredients. With the increase in the elderly population in the future, it is expected that the need to address insomnia while reducing healthcare costs will increase. In this regard, it is required to discover safe and inexpensive natural sleep-enhancing substances.

Gut microbiota (gut flora or microbiome) refers to the microorganisms that live in the human digestive tracts, including bacteria, archaea, fungi, etc. Various studies and statistics have confirmed that the gut microbiota is mainly composed of the following three enterotypes: *Prevotella, Bacteroides* and *Ruminococcus.*

Of these, the microorganisms in the genus *Prevotella* are primarily associated with carbohydrates and monosaccharides, while those in the genus *Bacteroides* are known to be associated with proteins, amino acids and saturated fats. Furthermore, one enterotype may dominate depending on the diet, and changing the diet causes a corresponding change in the number of species (Wu et al, "Linking Long-Term Dietary Patterns with Gut Microbial Enterotypes". Science, 2011). According to a study conducted in 2021, it was found that diet and exercise in childhood can have a significant impact on the overall composition and diversity of the microbiome in adulthood (Monica P. McNamara et al, "Early-life effects of juvenile Western diet and exercise on adult gut microbiome composition in mice", The Journal of Experimental Biology, 2021*).*

Therefore, researches to identify whether the composition and diversity of the gut microbiome, which are altered by the dietary and lifestyle factors, are associated with the development or progression of certain diseases are increasing.

However, researches on the relationship between gut microbes and sleep patterns in mammals, including human, or on natural sleep aids that utilize gut microbes are still insufficient. If a sleep aid that utilizes the gut microbes already present in the human body is developed, it could provide safer sleep for a long time without tolerance.

The matters described above as the background art are only for the purpose of improving the understanding of the background of the present disclosure, and should not be taken as an acknowledgment that they correspond to the prior art already known to those skilled in the art.

### [Disclosure]

### [Technical Problem]

The inventors of the present disclosure have made consistent efforts to develop a safer natural product-derived sleep aid. As a result, they have confirmed that *Prevotella histicola,* which is one of the human oral or intestinal microorganisms, or its culture is effective in increasing sleep duration and decreasing sleep latency and wake time, and have completed the present disclosure.

Accordingly, the present disclosure is directed to providing a novel *Prevotella histicola* strain.

The present disclosure is also directed to providing a composition for preventing, ameliorating or treating sleep disorder, which contains *Prevotella histicola* or a culture, lysate, extract or fermentation product thereof as an active ingredient.

The present disclosure is also directed to providing a composition for preventing, ameliorating or treating sleep disorder, which contains an extracellular vesicle derived from *Prevotella histicola* as an active ingredient.

The present disclosure is also directed to providing a method for preventing, ameliorating or treating sleep disorder, which includes a step of administering a composition containing *Prevotella histicola* or a culture, lysate, extract or fermentation product thereof to a subject.

The present disclosure is also directed to providing a method for preventing, ameliorating or treating sleep disorder, which includes a step of administering a composition containing extracellular vesicles derived *from Prevotella histicola* to a subject.

The present disclosure is also directed to providing a therapeutic use of *Prevotella histicola* or a culture, lysate, extract or fermentation product thereof.

The present disclosure is also directed to providing a therapeutic use of extracellular vesicles derived *from Prevotella histicola.*

The present disclosure is also directed to providing a method for preparing a composition for preventing, ameliorating or treating sleep disorder, which includes:
(a) a step of preparing a *Prevotella histicola* strain; and
(b) a step of culturing the strain in a culture medium.

The present disclosure is also directed to providing a method for preparing a composition for preventing, ameliorating or treating sleep disorder, which includes a step of preparing extracellular vesicles derived from a *Prevotella histicola* strain.

Other purposes and advantages of the present disclosure become more apparent by the following detailed description, claims and drawings.

### [Technical Solution]

According to an aspect of the present disclosure, the present disclosure provides a novel *Prevotella histicola* strain.

According to a specific exemplary embodiment of the present disclosure, the *Prevotella histicola* is isolated.

The *Prevotella histicola* strain is specifically isolated from the oral cavity or gastrointestinal tract, more specifically from oral saliva.

According to a specific exemplary embodiment of the present disclosure, the *Prevotella histicola* strain exhibits preventive, ameliorative or therapeutic activity in sleep disorder.

According to another aspect of the present disclosure, there is provided a composition for preventing, ameliorating or treating sleep disorder, which contains a *Prevotella histicola* strain or a culture, lysate, extract or fermentation product thereof as an active ingredient.

According to another aspect of the disclosure, there is provided a composition for preventing, ameliorating or treating sleep disorder, which contains extracellular vesicles derived *from Prevotella histicola* as an active ingredient.

In the present specification, the term "*Prevotella histicola*" or "*Prevotella histicola* strain" refers to a strain of the genus *Prevotella,* which are anaerobic, non-motile, Gram-negative bacilli that inhabit the human oral cavity or gastrointestinal tract. Specifically, the *Prevotella histicola* or *Prevotella histicola* strain may be *Prevotella histicola* deposited with an accession number selected from a group consisting of accession numbers KCCM13103P, KCCM13104P and KCCM13105P, although not being limited thereto.

More specifically, the *Prevotella histicola* may be selected from a group consisting of KCCM13103P (*Prevotella histicola* KCOM 3796), KCCM13104P *(Prevotella histicola* KCOM 4081) and KCCM13105P *(Prevotella histicola* KCOM 4227) deposited in the Korean Culture Center of Microorganisms on January 11, 2022.

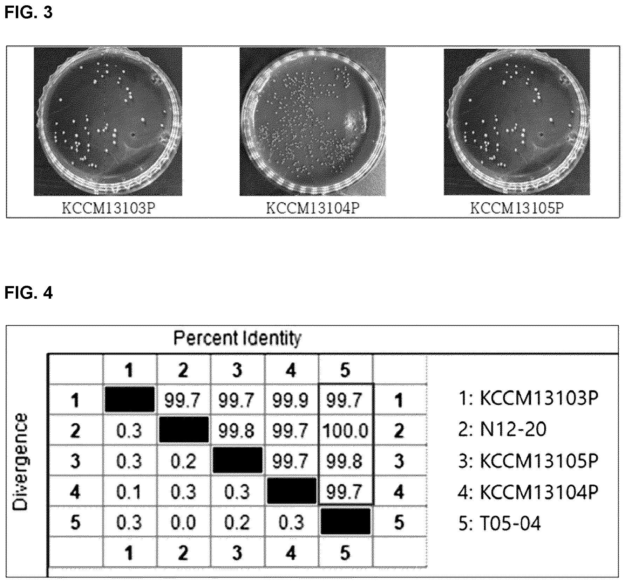

More specifically, said 16S rDNA sequences of *Prevotella histicola* comprise the 16S rDNA sequences of KCOM 3796, KCOM 4081, KCOM 4081, and KCOM 4227, which exhibit greater than 99% homology to the standard strain (T05-04) (FIG. 4).

According to a specific exemplary embodiment of the present disclosure, the 16S rDNA sequence of *Prevotella histicola* includes a sequence selected from a group consisting of SEQ ID NOS 3, 4 and 5.

The *Prevotella histicola* contained in the composition according to the disclosure may exist as live or dead bacteria, and may also be in a dried or freeze-dried form. The type of the bacteria suitable to be contained in the composition and the method for preparation thereof are well known to those skilled in the art. For example, the *Prevotella histicola* can be prepared into a culture obtained by cultivation in a known liquid or solid medium, a fermentation product obtained by cultivation of the strain with an additional ingredient, an extract obtained by extracting the strain with an organic solvent, a lysate (or homogenate) obtained by lysing or homogenizing the cell membrane of the strain, etc., although not being limited thereto.

In a specific exemplary embodiment, the composition may be a composition containing a *Prevotella histicola* strain existing as live or dead bacteria.

In another specific exemplary embodiment, the composition may be a composition containing a culture, lysate, extract or fermentation product of a *Prevotella histicola* strain.

In another specific exemplary embodiment, the composition may be a composition containing extracellular vesicles derived from *Prevotella histicola* or a culture, lysate, extract or fermentation product thereof.

Extracellular vesicles (EVs) enable the exchange of materials (proteins, lipids and genetic materials) between cells and function as signaling mediators both physiologically and pathologically. The extracellular vesicles are largely classified into exosomes and microvesicles. The exosomes vary in size depending on their origins. They are intraluminal vesicles formed by the inward retraction of the endosomal membrane during the maturation of multi-vesicular endosomes, and are secreted when the multi-vesicular endosomes bind to the cell surface. The microvesicles are vesicles of 50-1000 nm in size, which protrude from the plasma membrane and released out of the cell. Individual cells produce different extracellular vesicles depending on their physiological states and secrete extracellular vesicles having specific lipid/protein/nucleic acid compositions (Lee, Jae-Wook (2019). Shedding light on the cell biology of extracellular vesicles. *BRIC View* 2019-R03).

In the present specification, the term "extracellular vesicles" encompasses exosomes and microvesicles.

The exosomes or extracellular vesicles have various diameters in a range of about 1 to 1,000 nm. The *Prevotella histicola*-derived exosomes have a diameter of specifically 10-1,800 nm, most specifically 10-600 nm.

The exosomes or extracellular vesicles contained in the composition of the present disclosure are contained in large quantities in a culture (e.g., a supernatant of the culture) *Prevotella histicola.*

Since the exosomes or extracellular vesicles are contained in large quantities in a culture of *Prevotella histicola* (e.g., a culture supernatant), the exosomes or extracellular vesicles themselves may be isolated and purified for use as a therapeutic agent, or a culture, lysate, extract or fermentation product containing the exosomes or extracellular vesicles in large quantities may be utilized as a therapeutic agent.

In the present specification, the term "isolation" or "extraction" includes not only a process of selectively obtaining a desired substance (e.g., exosomes) from a biological sample (e.g., a *Prevotella histicola* culture) (positive isolation) but also a process of selectively removing impurities other than the desired substance (negative isolation). Therefore, the term "isolation" may be used with the same meaning as "obtainment", "extraction" or "purification". In the present disclosure, the isolation of exosomes or extracellular vesicles may be performed by any method commonly employed in the art without limitation. For example, a commercially available exosome isolation kit (e.g.,, EXO-BB, ExoQuick^{®}-ULTRA, ExoQuick^{®}-TC, Capturem^{™} exosome isolation kit, total exosome isolation kit, ExoTrap^{™} exosome isolation spin column kit, Exo2D^{™}, etc.) may be utilized, or separation based on the difference in the specific gravity of ingredients in a solution (e.g., centrifugation), separation based on size (e.g., ultrafiltration or vacuum filtration) or separation based on the affinity for a specific substrate (e.g., affinity chromatography) may be included. However, any separation method based on the intrinsic physical properties of a desired substance in a nonhomogeneous sample commonly used in the art may be used without limitation.

According to a specific exemplary embodiment of the present disclosure, the culture, lysate, extract or fermentation product of *Prevotella histicola* may contain vesicles, intraluminal vesicles (ILVs), endosomes and/or multi-vesicular endosomes (MVEs) in the cells of *Prevotella histicola.*

The intracellular vesicles may be secreted as exosomes through binding to the plasma membrane of *Prevotella histicola.*

According to a specific exemplary embodiment of the present disclosure, the culture, lysate, extract or fermentation product of *Prevotella histicola* may contain extracellular vesicles (EVs) of *Prevotella histicola* containing microvesicles and/or exosomes.

According to a specific exemplary embodiment of the present disclosure, the sleep disorder may be one or more selected from a group consisting of insomnia, breathing-related sleep disorder and circadian rhythm sleep disorder.

In the present specification, the term "insomnia" refers to trouble sleeping at night, which interferes with daytime activities.

Insomnia is largely classified into three types: sleep onset insomnia, or difficulty falling asleep; sleep maintenance insomnia, or frequent awakenings during sleep and difficulty falling asleep again; and sleep terminal insomnia, or waking up very early in the morning and difficulty falling asleep again.

In the present specification, the term "breathing-related sleep disorder" refers to a condition wherein drowsiness or insomnia is caused by breathing disorder during sleep. Difficulty breathing regularly or pause in breathing occurs during sleep due to breathing disorder, resulting in wake-up. The main symptom is excessive sleepiness, which is caused by frequent waking up during nighttime sleep due to abnormalities in breathing.

There are three main types of breathing disorder occurring in breathing-related sleep disorder. The first is obstructive sleep apnea syndrome, which is characterized by recurrent episodes of apnea or decreased breathing due to airway obstruction during sleep and is the most common type. The second is central sleep apnea syndrome, where there is no airway obstruction but apnea occurs during sleep due to neurological or heart diseases. Lastly, central alveolar hypoventilation syndrome is a phenomenon where oxygen level in the arteries decreases due to impaired respiratory control.

In the present specification, the term "circadian rhythm sleep disorder" refers to a condition characterized by recurring episodes of drowsiness or insomnia caused by changes in the sleep-wake cycle. In other words, it is characterized by recurrent and persistent excessive sleepiness or insomnia due to a mismatch between the sleep-wake cycle required by the environment (e.g., night work, foreign travel, etc.) and the individual's circadian sleep-wake cycle.

The circadian rhythm sleep disorder is categorized into four types. The first type is the delayed sleep phase type, in which an individual's sleep-wake cycle is delayed compared to what is socially required. The second type is the advanced sleep phase type, in which people tend to go to bed and wake up much earlier as compared to normal individuals. It is common in older populations. The third type is the jet lag type, in which traveling abroad causes a mismatch between an individual's sleep-wake cycle and the sleep-wake cycle required by the new time zone. The fourth type is the shift work type, in which there is a mismatch between the sleep-wake cycle required by shift work and the individual's sleep-wake cycle.

According to a specific exemplary embodiment of the present disclosure, the prevention, amelioration or treatment of sleep disorder is achieved by increasing total sleep duration.

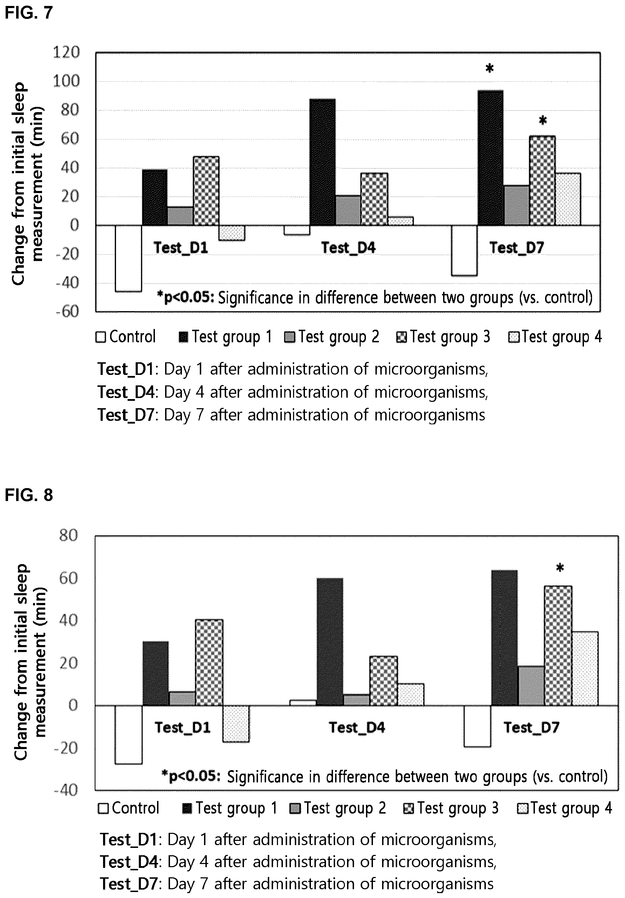

According to an example of the present disclosure, a group to which the *Prevotella histicola* of the present disclosure was administered exhibited increase in total sleep duration (recorded for 24 hours) as compared to a control group to which *Prevotella stercorea* was administered (FIG. 7).

According to a specific exemplary embodiment of the present disclosure, the prevention, amelioration or treatment of sleep disorder is achieved by increasing non-REM (NREM) sleep duration.

According to an example of the present disclosure, a group to which the *Prevotella histicola* of the present disclosure was administered showed increase in non-REM sleep duration as compared to a control group to which *Prevotella stercorea* was administered (FIG. 8).

According to a specific exemplary embodiment of the present disclosure, the prevention, amelioration or treatment of sleep disorder is achieved by increasing REM sleep duration.

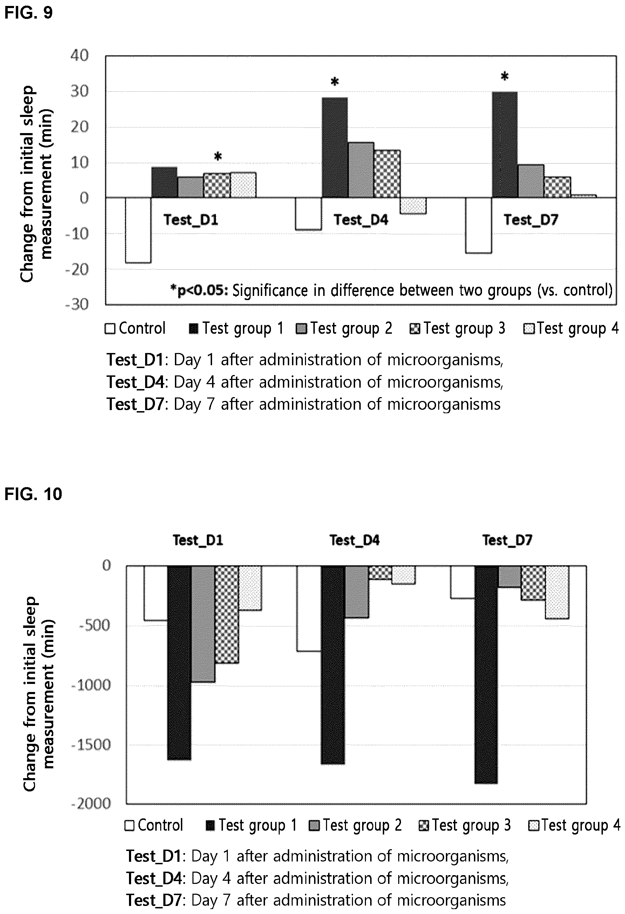

According to an example of the present disclosure, a group to which the *Prevotella histicola* of the present disclosure was administered showed increase in REM sleep duration as compared to a control group to which *Prevotella stercorea* was administered (FIG. 9).

According to a specific exemplary embodiment of the present disclosure, the prevention, amelioration or treatment of sleep disorder is achieved by reducing sleep latency.

According to an example of the present disclosure, a group to which the *Prevotella histicola* of the present disclosure was administered showed overall reduction in sleep latency as compared to a control group to which *Prevotella stercorea* was administered (FIG. 10).

According to a specific exemplary embodiment of the present disclosure, the prevention, amelioration or treatment of sleep disorder is achieved by reducing total wake time.

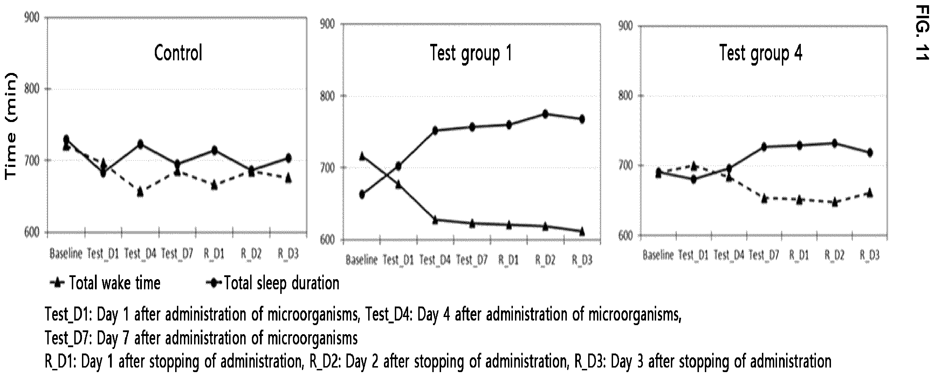

According to an example of the present disclosure, a group to which the *Prevotella histicola* of the present disclosure was administered showed overall reduction in in wake time as compared to a control group to which *Prevotella stercorea* was administered (FIG. 11).

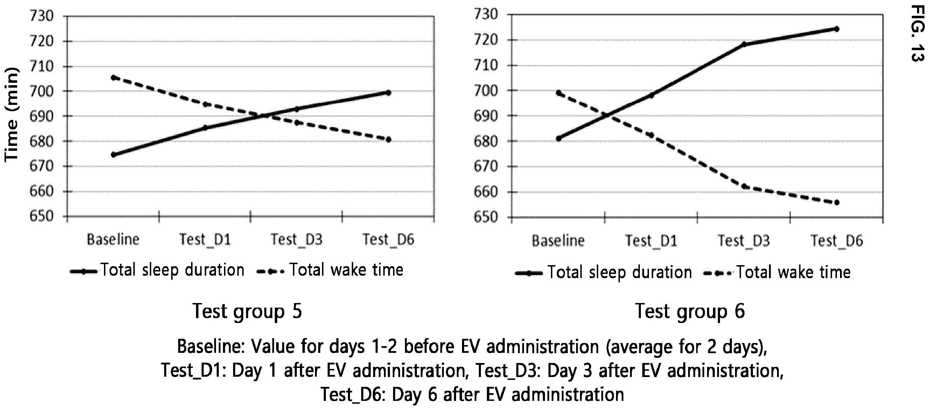

According to an example of the present disclosure, extracellular vesicles derived from the *Prevotella histicola* strain of the present disclosure exhibited increase in total sleep duration and decreased total wake time (FIG. 13).

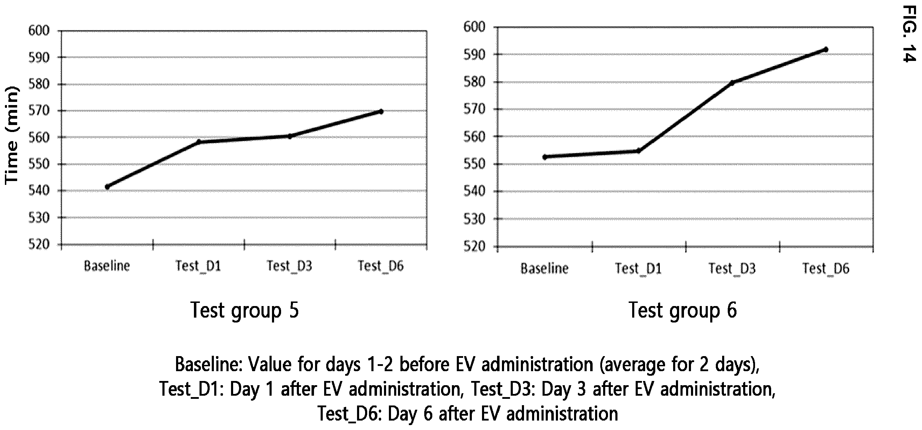

According to an example of the present disclosure, extracellular vesicle from the *Prevotella histicola* strain of the present disclosure exhibited increased non-REM sleep duration (FIG. 14).

According to a specific exemplary embodiment of the present disclosure, the composition is a pharmaceutical composition.

The present disclosure also provides a method for preventing, ameliorating or treating sleep disorder, which includes administering a therapeutically effective amount of a *prevotella histicola,* a culture, lysate, extract or fermentation product thereof; or extracellular vesicles derived from the *Prevotella histicola* or a culture, lysate, extract or fermentation product thereof to a subject in need thereof.

According to another aspect of the present disclosure, the present disclosure provides a therapeutic use of *Prevotella histicola* or a culture, lysate, extract or fermentation product thereof.

According to another aspect of the disclosure, the present disclosure provides a therapeutic use of extracellular vesicle derived from *Prevotella histicola* or a culture, lysate, extract or fermentation product thereof.

According to a specific exemplary embodiment of the present disclosure, the therapeutic use is for treatment of sleep disorder.

As used herein, the "subject" refers to a mammal which is the subject of treatment, observation or experimentation. Specifically, it may be a human or animal in need of prevention, amelioration and/or treatment of sleep disorder.

The pharmaceutical compositions according to the present disclosure may be administered orally or parenterally.

For parenteral administration, it may be administered, for example, by intravenous injection, transdermal administration, subcutaneous injection, intramuscular injection, intravitreal injection, eye drop administration, intracerebroventricular injection, intrathecal injection, intrathecal injection, intraamniotic injection, intraarterial injection, intraarticular injection, intracardiac injection, intracavernous injection, intracerebral injection, intracisternal injection, intracoronary injection, intracranial injection, intradural injection, epidural injection, intrahippocampal injection, intranasal injection, intraosseous injection, intraperitoneal injection, intrapleural injection, intraspinal injection, intrathoracic injection, intrathymic injection, intrauterine injection, intravaginal injection, intraventricular injection, intravesical injection, subconjunctival injection, intratumoral injection, topical injection, etc.

The pharmaceutical composition of the present disclosure may contain a pharmaceutically acceptable carrier. In the present specification, the term "pharmaceutically acceptable carrier" means a carrier or diluent which does not significantly stimulate an organism and does not inhibit the biological activity and properties of the administered ingredient. In the present specification, saline, sterile water, Ringer's solution, buffered saline, dextrose solution, maltodextrin solution, glycerol, ethanol and mixtures of one or more of these components may be used as the pharmaceutically acceptable carrier. If necessary, common additives such as antioxidants, buffers, bacteriostatic agents, etc. may be added for formulation into an injection suitable for injection into a tissue or organ. In addition, an isotonic sterile solution or, in some cases, a dry preparation (particularly a lyophilized preparation) that can be made into an injectable solution by adding sterile water or normal saline may be prepared. Furthermore, target organ-specific antibodies or other ligands can be used in combination with the carriers for specific action on the target organ. Suitable preparations known in the art may be referred to from those disclosed in the literature (Remington's Pharmaceutical Science, Mack Publishing Company, Easton PA).

Specifically, the composition of the present disclosure may further contain a filler, an excipient, a disintegrant, a binder, a glidant, etc. Further, the composition of the present disclosure may be formulated using methods known in the art to provide rapid, sustained or delayed release of the active ingredient after administration to a mammal.

In the present specification, the "administration" may refer to introducing the composition of the present disclosure into a subject by any suitable means, and the administration of the composition of the present disclosure may be achieved via a variety of oral or parenteral routes, as long as it can reach the target tissue.

For example, the composition of the present disclosure may be administered by intramuscular, intravenous or intraperitoneal injection.

For injection, it may specifically be formulated into a pharmacologically appropriate buffer such as Hank's solution, Ringer's solution or physiological saline buffer. For transmucosal administration, a non-penetrating agent suitable for the barrier to be crossed is used in the formulation. Such non-penetrating agents are generally known in the art.

Formulations for parenteral administration include a sterile aqueous solution, a non-aqueous solution, a suspension, an emulsion, etc. For the non-aqueous solution or suspension, propylene glycol, polyethylene glycol, vegetable oil such as olive oil, an injectable ester such as ethyl oleate, etc. may be used.

In the present specification, the "effective amount" means an amount necessary to delay or completely stop the onset or progression of a particular disease to be treated, and the effective amount of *Prevotella histicola* contained in the pharmaceutical composition of the present disclosure means an amount necessary to achieve the effect of preventing, ameliorating or treating sleep disorder. Accordingly, the effective amount may be adjusted depending on the type of the disease, the severity of the disease, the type and content of other ingredients in the composition and various other factors, including the patient's age, body weight, general health, sex and diet, administration time, administration route, duration of treatment, and concomitantly used medications. It will be obvious to those skilled in the art that the appropriate total daily dosage can be determined by a physician within the scope of sound medical judgment.

For the purpose of the present disclosure, the specific therapeutically effective amount for a particular patient will specifically vary depending on various factors including the type and degree of response to be achieved, the age, body weight, general health status, sex and diet of a patient, administration time, administration route, the excretion rate of the composition, the duration of treatment, the drugs used concurrently with the specific composition, and similar factors well known in the field of medicine.

In the present specification, the "treatment" means an approach to achieve a beneficial or desirable clinical outcome. For the purpose of the present disclosure, the beneficial or desirable clinical outcome includes, without limitation, alleviation of symptoms, reduction in disease range, stabilization of a disease state (i.e., stopping of worsening), delay or reduction in the rate of disease progression, and amelioration or temporary alleviation and mitigation of a disease state (partial or overall), whether detectable or undetectable. In addition, the "treatment" can also mean increased survival rate as compared to the expected survival rate without the treatment. The "treatment" refers to both therapeutic treatment and preventive or prophylactic measure. These treatments include not only those required for disorders to be prevented but also those required for disorders that have occurred already. The "amelioration" or "alleviation" of a disease means reduction in the extent of a disease state and/or undesirable clinical signs and/or slowing or lengthening of the time course of progression, as compared to the absence of treatment.

According to a specific exemplary embodiment of the present disclosure, the composition of the present disclosure is a food composition.

*Prevotella histicola,* a culture, lysate, extract or ferment thereof, or extracellular vesicles derived from *Prevotella histicola* or a culture, lysate, extract or fermentation product thereof contained in the food composition of the present disclosure are the same as described above.

When the composition of the present disclosure is utilized as a food composition, the food composition may be in the form of a health functional food, a condiment, a beverage, a bar, etc. Furthermore, the food composition containing the strain as an active ingredient may include a beverage such as fermented milk, etc. Accordingly, the present disclosure provides a lactic acid bacteria starter for food fermentation containing *Prevotella histicola* or a culture thereof.

The food composition of the present disclosure may be prepared using a sitologically suitable and physiologically acceptable adjuvant in addition to the active ingredient described above. The adjuvant may include an excipient, a disintegrant, a sweetener, a binder, a coating agent, an extender, a lubricant, a glidant, a flavoring agent, etc.

The food composition may specifically be formulated as a food composition containing one or more sitologically acceptable carrier in addition to the active ingredient described above.

For example, for formulation into a tablet or a capsule, the active ingredient may be combined with an oral, non-toxic, pharmaceutically acceptable inert carrier such as ethanol, glycerol, water, etc. Also, if desired or necessary, a suitable binder, lubricant, disintegrant and colorant or a mixture thereof can be included in the formulation. The suitable binder is not limited to but includes starch, gelatin, a natural sugar such as glucose or beta-lactose, a corn-based sweetener, a natural or synthetic gum such as acacia, tragacanth or sodium oleate, sodium stearate, magnesium stearate, sodium benzoate, sodium acetate, sodium chloride, etc. The disintegrant is not limited to but includes starch, methyl cellulose, agar, bentonite, xanthan gum, etc. In a composition formulated as a liquid solution, one or more of saline, sterile water, Ringer's solution, buffered saline, albumin injection solution, dextrose solution, maltodextrin solution, glycerol and ethanol may be used as an acceptable pharmaceutical carrier that is suitable for sterilization and biologically compatible. If necessary, other common additives such as an antioxidant, a buffer, a bacteriostat, etc. can be added. In addition, a diluent, a dispersant, a surfactant, a binder, a lubricant, etc. can be additionally added to formulate an injectable formulation such as an aqueous solution, a suspension, an emulsion, etc., a pill, a capsule, a granule or a tablet.

The food composition according to the present disclosure can be added to various food products. The food products to which the composition of the present disclosure can be added include, for example, beverages, vitamin complexes, dietary supplements, etc.

The food composition of the present disclosure may contain ingredients customarily added when preparing food products. For example, it may contain a protein, a carbohydrate, a fat, a nutrient, a condiment and a flavoring agent. Examples of the carbohydrate include: common sugars such as monosaccharides, e.g., glucose, fructose, etc.; disaccharides, e.g., maltose, sucrose, etc.; oligosaccharides; polysaccharides, e.g., dextrin, cyclodextrin, etc., and sugar alcohols such as xylitol, sorbitol, erythritol, etc. As the flavorant, natural flavorants [thaumatin or stevia extract (e.g., rebaudioside A, glycyrrhizin, etc.)] and synthetic flavorants (saccharin, aspartame, etc.) can be used. For example, if the food composition of the present disclosure is prepared into a beverage or a drink, it may further contain citric acid, fructose syrup, sugar, glucose, acetic acid, malic acid, fruit juice, various plant extracts, etc.

In accordance with another aspect of the present disclosure, there is provided a feed additive or a feed containing *Prevotella histicola* or a culture, lysate, extract or fermentation product thereof; or extracellular vesicles derived from the *Prevotella histicola* or a culture, lysate, extract or fermentation product thereof as an active ingredient.

When utilized as a feed additive, the composition may be a 20-90% concentrate or formulated as a powder or a granule. The feed additive may further comprise one or more of the followings: an organic acid such as citric acid, fumaric acid, adipic acid, lactic acid, malic acid, etc.; a phosphate such as sodium phosphate, potassium phosphate, acid pyrophosphate, polyphosphate, etc.; or a natural antioxidant such as polyphenol, catechin, alpha-tocopherol, rosemary extract, vitamin C, green tea extract, licorice extract, chitosan, tannic acid, phytic acid, etc. When utilized as a feed, the composition may be formulated in the form of a conventional feed and may contain conventional feed ingredients.

The feed additive and feed may further contain cereals such as milled or shredded wheat, oats, barley, corn and rice; vegetable protein feeds such as feeds based primarily on rape, soybean and sunflower; animal protein feeds such as blood meal, meat meal, bone meal and fish meal; sugars; and dairy products such as dry ingredients containing various milk powders and whey powders. In addition, it may further contain nutritional supplements, digestion and absorption enhancers, growth promoters, etc.

The feed additive may be administered to an animal either alone or in combination with another feed additive in an edible carrier. Furthermore, the feed additive can be readily administered to an animal as a top dressing or by mixing with an animal feed, or as an oral formulation separately from the feed. When administered separately from the animal feed, the feed additive can be formulated into an immediate-release or sustained-release formulation in combination with a sitologically acceptable edible carrier as is well known in the art. The edible carrier can be a solid or a liquid, such as cornstarch, lactose, sucrose, bean flake, peanut oil, olive oil, sesame oil and propylene glycol. When a solid carrier is used, the feed additive may be a tablet, a capsule, a powder, a troche, a sugar-containing tablet, or a top dressing in a microdispersed form. When a liquid carrier is used, the feed additive may be in the form of a soft gelatin capsule, a syrup, a suspension, an emulsion or a solution.

Furthermore, the feed additive and feed may contain an adjuvant, e.g., a preservative, a stabilizer, a wetting or emulsifying agent, a solubilizer, etc. The feed additive may be added to an animal feed by dipping, spraying or mixing.

The feed or feed additive of the present disclosure can be provided to a number of animals, including mammals, poultry and fish.

The mammals include, but are not limited to, pig, cattle, sheep, goat and laboratory rodents, as well as pets (e.g., dog and cat). The poultry include, but are not limited to, chicken, turkey, duck, goose, pheasant, quail, etc. And, the fish include, but are not limited to, trout, etc.

The feed or feed additive of the present disclosure can be provided to animals to enhance animal growth, immunity, etc.

The amount of the *Prevotella histicola* strain contained in the composition according to the present disclosure may be about 10⁶ to 10¹² CFU/mL, e.g., 10⁷ to 10¹¹ CFU/mL or 10⁸ to 10¹¹ CFU/mL, based on a single dose. Specifically, the strain may be administered as live bacteria. It may also be administered after being killed or attenuated. In addition, when a culture supernatant is used for preparation, it may be further sterilized by heat treatment. The amount of the strain required to achieve minimal efficacy and the daily dose may vary depending on the physical or medical condition of a person consuming the strain, but may generally be about 10⁶ to 10¹² CFU/mL, e.g., 10⁷ to 10¹¹ CFU/mL, 10⁸ to 10¹¹ CFU/mL or 10⁸ to 10¹¹ CFU/mL.

The amount of extracellular vesicles derived from the *Prevotella histicola* strain contained in the composition according to the disclosure may be about 10⁶ to 10¹² CFU/mL, e.g., 10⁷ to 10¹¹ CFU/mL or 10⁸ to 10¹¹ CFU/mL, based on a single dose.

According to another aspect of the present disclosure, there is provided a method for preparing a composition for preventing, ameliorating or treating sleep disorder, which includes:
(a) a step of preparing a *Prevotella histicola* strain; and
(b) a step of culturing the strain in a culture medium.

According to a specific exemplary embodiment of the present disclosure, the method may further include: (c) a step of isolating extracellular vesicles from the culture medium.

According to another aspect of the present disclosure, there is provided a method for preparing a composition for preventing, ameliorating or treating sleep disorder, which includes a step of preparing extracellular vesicles derived from a *Prevotella histicola* strain.

### [Advantageous Effects]

The features and advantages of the present disclosure are summarized as follows:
(i) The present disclosure provides a novel *Prevotella histicola* strain.
(ii) The *Prevotella histicola* strain of the present disclosure can be used as a safe drug or a food product for preventing and treating sleep disorder by increasing total sleep duration, non-REM (NREM) sleep duration, and REM sleep duration, as well as reducing sleep latency and wake time.

### [Brief Description of Drawings]

FIG. 1 is a schematically describes the entire experimental process.
FIG. 2 shows the photograph of a sleep electroencephalography system.
FIG. 3 shows the photographs of cultured deposited strains.
FIG. 4 shows a result of 16S rDNA nucleotide sequence homology (%) analysis of cultured deposited strains.
FIG. 5 shows a result of 16S rDNA nucleotide sequence ANI value analysis of cultured deposited strains.
FIG. 6 shows a result of phylogenetic analysis of cultured deposited strains.
FIG. 7 shows a result of comparing the change in sleep duration (recorded for 24 hours) as compared to the initially measured sleep duration (recorded for 24 hours) by sleep electroencephalography.
FIG. 8 shows a result of comparing the change in non-REM sleep duration (recorded for 24 hours) as compared to the initially measured non-REM sleep duration (recorded for 24 hours) by sleep electroencephalography.
FIG. 9 shows a result of comparing the change in REM sleep duration as compared to the initially measured REM sleep duration (recorded for 24 hours) by sleep electroencephalography.
FIG. 10 shows a result of comparing the change in sleep latency (recorded for 24 hours) as compared to the initially measured total sleep latency (time taken to fall asleep) by sleep electroencephalography.
FIG. 11 shows a result of investigating the change in sleep duration and wake time (recorded for 24 hours) of a control group, a test group 1 and a test group 4 by sleep electroencephalography.
FIG. 12 shows a result of investigating the change in non-REM sleep duration (recorded for 24 hours) of different groups by sleep electroencephalography.
FIG. 13 shows a result of investigating the change in sleep duration and wake time as measured by sleep electroencephalography (recorded for 24 hours).
FIG. 14 shows a result of investigating the change in non-REM sleep duration (recorded for 24 hours) as measured by sleep electroencephalography.

### [Best Mode]

Hereafter, the present disclosure will be described in more detail through examples. These examples are intended solely to explain the present disclosure in more detail, and it will be obvious to those having common knowledge in the art that the scope of the present disclosure is not limited by the examples.

### Examples

### Materials and Methods

### 1. Experimental animals

7-week-old Sprague-Dawley albino rats were used for experiment. All animals were acclimatized to a light/dark cycle of 09:00-21:00 for 1 week at constant temperature (20-21 °C). Water and feed were provided ad libitum.

### 2. Preparation of microorganisms

### 2-1. Source of deposited strains

Three deposited strains (KCCM13103P, KCCM13104P and KCCM13105P) were isolated from the oral saliva of three patients who visited the Department of Periodontics of the Chosun University Dental Hospital. The information of the deposited strains is shown in Table 1. Personal information that could identify the patients was anonymized and only the information described in Table 1 was provided to researchers.

**[Table 1]**

| **Information about deposited strains** | | | | | | |
|---|---|---|---|---|---|---|
| Accession number | Strain name | Strain source information | | | | |
| | | Sex | Age | Source of isolation | Diagnosis | Date of isolation |
| KCCM13103P (KCOM 3796) | *Prevotella histicola* | F | 67 | Saliva | Peri-implantitis | 2019.10.22 |
| KCCM13104P (KCOM 4081) | *Prevotella histicola* | F | 62 | Saliva | Chronic periodontitis | 2020.08.05 |
| KCCM13105P (KCOM 4227) | *Prevotella histicola* | F | 82 | Saliva | Chronic periodontitis | 2020.10.21 |

### 2-2. Culturing of microorganisms

The patient's saliva was diluted 1,000 and 10,000-fold in phosphate buffered saline (1x PBS) and plated on a blood agar medium [tryptic soy broth (TSB, Becton Dickinson, Franklin Lakes, NJ, USA) supplemented with 0.05% cysteine HCl-H₂O, 0.5% yeast extract, 5 µg/mL hemin, 2 µg/mL vitamin K₁ and 5% sheep blood]. After culturing for 7 days in an anaerobic bacteriological incubator (Bactron I, Sheldon Manufacturing Inc, Cornelius, OR, USA) at 37 °C, the grown bacterial colonies were selected randomly and then isolated.

### 2-3. Identification of microorganisms

The isolated microorganisms were identified by 16S rDNA nucleotide sequencing and whole genome nucleotide sequencing. To analyze the 16S rDNA nucleotide sequences of the microorganisms, 16S rDNA was amplified using universal PCR primers (27F; 5'-AGA GTT TGA TCM[A/C] TGG CTC AG-3' (SEQ ID NO 1), 1492R; 5'-TAG GGY[C/T] TAC CTT GTT ACG ACT T-3' (SEQ ID NO 2)), Accupomer^{®} Premix (Bioneer, Seoul, Korea) and a PTC-200 polymerase chain reaction (PCR) machine (MJ Research INC., Watertown, MA, USA). A PCR reaction solution was prepared by adding 20 pmol of 27F and 1492R primers and 100 pg of bacterial genomic DNA to a final volume of 20 µL. PCR was conducted as follows. After initial denaturation at 94 °C for 2 minutes, followed by 30 cycles of denaturation at 94 °C for 1 minute, annealing at 55 °C for 1 minute and extension at 72 °C for 1 minute, extension was performed finally at 72 °C for 10 minutes. 2-µL aliquots of the final reaction solution were taken and subjected to electrophoresis on a 1.5% agarose gel to confirm the amplification. The amplified 16S rDNA was cloned into a pGEM-T easy vector (Promega, Madison, WI, USA) according to the manufacturer's instructions and sent to Bioneer (Daejeon, Korea) for nucleotide sequencing. The analyzed 16S rDNA sequences of KCCM13103P (KCOM 3796), KCCM13104P (KCOM 4081) and KCCM13105P (KCOM 4227) are shown as SEQ ID NOS 3 to 5.

To identify the deposited strains, the 16S rDNA nucleotide sequence homology of the deposited strains was analyzed against the 16S rDNA nucleotide sequence information of the standard *Prevotella histicola* strain (T05-04) and reference strain (N2-20) downloaded from Genbank (https://www.ncbi.nim.nih.gov/). After sequence alignment using the Clustal W algorithm method in the MegAlin program (version 7.0, DNAStar Lasergene, DNAStar Inc., Madison, WI, USA), the percent identity and divergence values of the standard, reference and deposited strains were calculated. The phylogenetic analysis result was depicted as a phylogenetic tree. In addition, the homology of the whole genome nucleotide sequences of the standard and deposited strains was analyzed using the USEARCH algorithm in the OrthoANI program (Lee et al, Int J Syst Evol Microbiol, 2016; 66: 1100-1103 and https://www.ezbiocloud.net/) to calculate the average nucleotide identity (ANI) values. When the bacteria are identified to the species level based on the bacterial whole genome nucleotide sequences, a target strain is determined as the same species as the reference strain if the homology of the 16S rDNA nucleotide sequence of the reference strain and the target strain is greater than 98.6% and the ANI value of the whole genome nucleotide sequence is greater than 95% (Chun et al. Int J Syst Evol Microbiol. 2018; 68: 461-466).

### 2-4. Preparation of deposited strain samples

5-10 colonies of the deposited *Prevotella histicola* strain activated by culturing in a solid medium in two 14-mL tubes containing 7 mL of a liquid medium were inoculated and cultured in an anaerobic bacteriological incubator at 37 °C for 24 hours. After adding 200 mL of a liquid medium to twelve 250-mL bottles and inoculating with 1 mL of the bacterial culture, culturing was performed for 24-28 hours. The bacterial culture was collected in a 250-mL centrifuge tube and centrifuged at 7000 rpm for 15 minutes. After discarding the supernatant, bacterial pellets were suspended in 3 mL of a PBS buffer and combined to a single centrifuge tube. Then, after dividing the same into 3 mL portions in 15-mL conical tubes, they were sealed with sterile cotton, frozen at -80 °C and lyophilized for 30-32 hours. To prepare the sample volume required for animal experiments, 10 µL of the bacterial suspension was taken from one tube, diluted 10⁴, 10⁵ or 10⁶ times with PBS buffer, and 10 µL of the diluted bacterial suspension was taken and plated on a solid medium. Then, the number of colonies was counted after culturing for 2-3 days. The lyophilized bacteria were placed in an anaerobic system pack (BD Gaspak EZ^{™} Pouch, USA) and stored at 4 °C to maintain the anaerobic condition of the bacteria until animal experiment.

### 3. Design of animal experiment using live strains

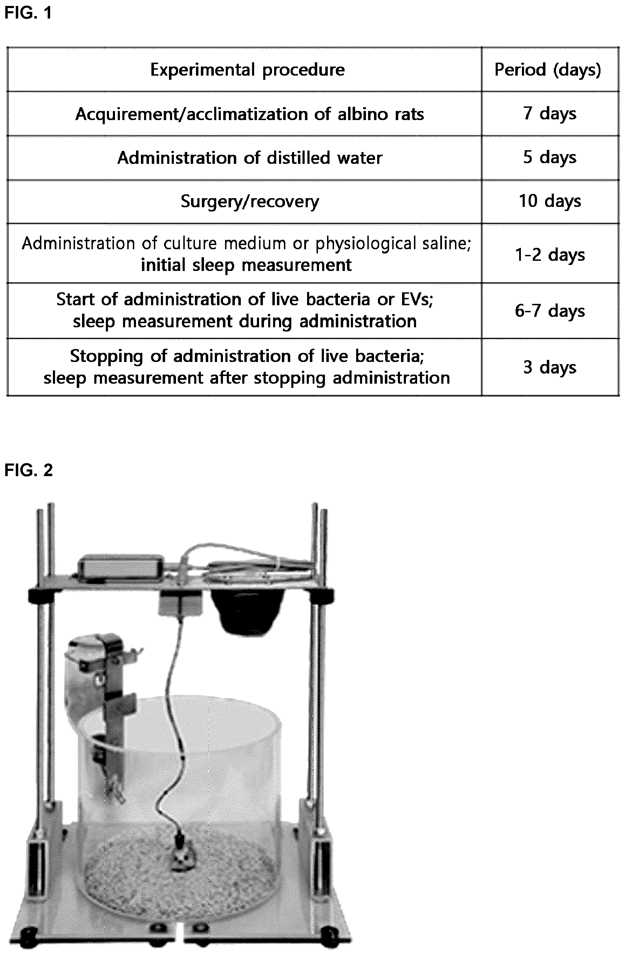

Animal experiment was performed using live strains for a control group (n = 5) and four test groups, i.e., a test group 1 (n = 5), a test group 2 (n = 5), a test group 3 (n = 4) and a test group 4 (n = 6). The overall experimental process is shown in FIG. 1. The control group was administered with a sample of *Prevotella stercorea* (*a* microorganism found in the human body; the second most prevalent species in the genus *Prevotella*)*.* The test group 1 was administered with a sample of the representative strains of *Prevotella histicola* (KCTC 15171; CCUG 55407; DSM 19854; JCM 15637). Another strain of *Prevotella histicola* (CCUG 60372) was administered to the test group 2, and *Prevotella histicola* sample collected from the oral cavities of Koreans were administered orally to the test groups 3 and 4 (KCCM13103P for the test group 3 and KCCM13105P for the test group 4). For acclimatization to oral administration, 1 mL of distilled water was administered orally at the same time each day (between 08:00 and 09:00) from 5 days prior to the start of sleep measurements. The control and test groups 1 and 2 were administered orally with 1 mL of a medium between 08:00 and 09:00 for 2 days for baseline sleep measurements prior to the administration of the cultured bacteria. For the test groups 3 and 4, lyophilized live bacteria samples were prepared without a medium and diluted in distilled water, which were administered orally between 08:00 and 09:00. Baseline sleep measurements were performed for 1-2 days prior to the administration of the microorganisms. The microorganisms used in each test group were genetically analyzed (16S rDNA sequencing) for confirmation, and the live bacterial count in the daily dose sample was 1×10¹⁰ CFU.

### 4. Design of animal experiment using extracellular vesicles (EVs) of strain

Animal experiment using EVs was performed for two test groups with four animals in each group. EV samples of the representative strains of *Prevotella histicola* (KCTC 15171; CCUG 55407; DSM 19854; JCM 15637) were administered orally to a test group 5, and EV samples of *Prevotella histicola* collected from the oral cavity of Koreans (KCCM13105P) were administered to a test group 6. For acclimatization to oral administration, 1 mL of distilled water was administered orally at the same time each day (between 08:00 and 09:00) from 5 days prior to the start of sleep measurements. The amount of the EV sample administered orally daily was 4.56 mg (extracted from 1×10⁹ CFU of live bacteria) for the group 5 and 2 mg for the group 6.

### 5. Measurement of sleep EEG

Electroencephalography (EEG 2 channel) and electromyography (EMG 1 channel) were performed (8239 Headmount, Pinnacle Technology Inc.) to analyze the sleep of the albino rats. Surgery was performed to implant a device into the skull for testing. After inducing deep anesthesia (using isoflurane), the albino rat's hair was shaved with an electric cutter. The head was clamped in a stereotaxic device and the scalp and subcutaneous connective tissue were incised together with a scalpel to expose the skull. After forming four holes on the skull using a dental drill, EEG electrodes were inserted for EEG measurement and EMG electrodes were inserted into two areas of the neck muscles for EMG measurements and then fastened using non-absorbable thread and dental acrylic. After suturing and disinfecting the incised site, antibiotics were administered intraperitoneally for three days to prevent inflammation from the surgery. After a minimum recovery period of 10 days, during which the wound was cleaned after surgery, sleep electroencephalography measurement was started. EEG and EMG recordings were started at 9 am and continued for 24 hours. For sleep stage analysis, the albino rat was placed in a cage and connected to a preamplifier. Then, sleep was recorded using a 3-channel EEG/EMG system (Sirenia Sleep Pro, Pinnacle Technology, Oregon ST, USA) of Pinnacle Technology Inc. (FIG. 2). Sleep stages were analyzed by dividing wakefulness, non-REM (non-rapid eye movement, deep sleep) and REM (rapid eye movement, light sleep) sleep stages depending on the amplitude values of the EEG and EEG waveforms. The EEG acquisition condition was set to a sampling rate of 200 Hz and a preamplification gain of 10. The channel acquisition condition was set to a gain of 1 and a filter Hz of 25 for EEG, and a gain of 1 and a filter Hz of 100 for EMG.

### Results

### 1. Culturing and identification of deposited bacteria

The cultured deposited strains are shown in FIG. 3.

*As* shown in FIG. 4, the homology (%, red box) between the deposited strains (KCCM13103P, KCCM13104P and KCCM13105P) and the *Prevotella histicola* standard strain (T05-04) and the reference strain (N2-20) was 99% or higher, indicating that the deposited strains are the same species as the standard and reference strains.

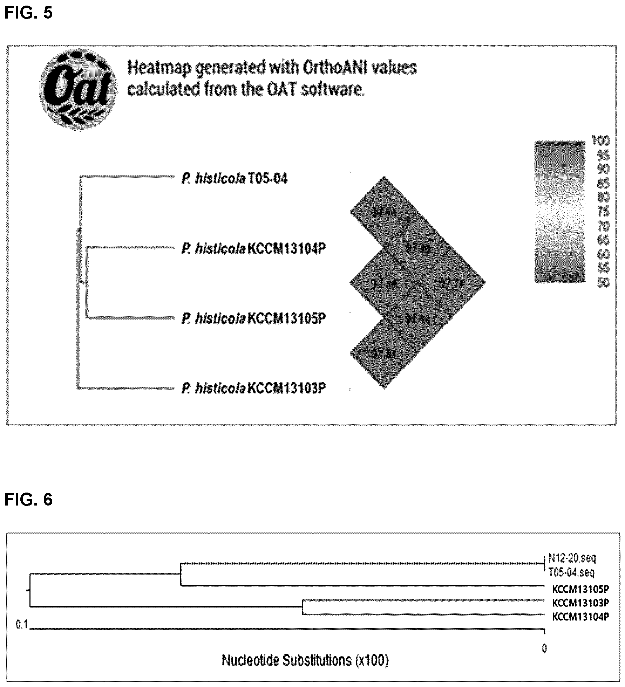

FIG. 5 shows the analysis result using the OrthoANI program. The ANI values between the standard strain (T05-04) and the three deposited strains were 95% or higher, indicating that all the deposited strains are *Prevotella histicola.*

As shown in the phylogenetic tree in FIG. 6, of the three deposited strains, KCCM13105P was the closest in evolutionary aspect to the *Prevotella histicola* standard strain (T05-04) and the reference strain (N2-20), followed by the two deposited strains, KCCM13103P and KCCM13104P.

### 2. Result of animal experiment using live strains

Sleep EEG results were obtained for all the experimental albino rats. Initial sleep measurements were taken on days 1 and 2 (measurement results were averaged), followed by sleep measurements on days 1, 4 and 7 after the administration of the microorganisms (for all groups) and sleep measurements on days 1 to 3 after the stopping of the administration of the microorganisms (measured for the control, test group 1 and test group 4 only). The comparison between the control and test groups was calculated by comparing the change from the initial sleep measurements to the later sleep measurements (later sleep measurements - initial sleep measurements). Statistical test was performed by the Mann-Whitney U test, which is a non-parametric statistical method, and p < 0.05 was considered significant for two-tailed tests.

FIG. 7 shows a result of comparing the change in sleep duration (recorded for 24 hours) as compared to the initially measured sleep duration (recorded for 24 hours) by sleep electroencephalography. In general, the test groups showed gradual increase in sleep duration as compared to the initial sleep measurements after the administration of the microorganisms, while the control group showed little change or slight decrease in sleep duration. In particular, sleep duration was increased by 1 hour or more on day 7 after the administration of the microorganisms in the test group 1 and the test group 3 as compared to before the administration, as compared to the control group (p < 0.05). The test group 2 and the test group 4 also showed increase in sleep duration on day 7 after the administration of the microorganisms as compared to before the administration, although it was not statistically significant.

To compare the change in sleep quality, the duration of non-REM sleep, which is a particularly deep state of sleep, was analyzed. FIG. 8 shows a result of comparing the change in non-REM sleep duration (recorded for 24 hours) as compared to the initially measured non-REM sleep duration (recorded for 24 hours) by sleep electroencephalography. There was a significant increase (p < 0.05) in non-REM sleep duration on day 7 after the administration of the microorganisms in the test group 3. The other test groups also showed increase, while the control group showed slight decrease. In particular, the test group 3 and the test group 1 showed increase in non-REM sleep duration of about 1 hour on day 7 after the administration of the microorganisms, indicating that sleep quality was improved significantly.

FIG. 9 shows a result of comparing the change in REM sleep duration as compared to the initially measured REM sleep duration (recorded for 24 hours) by sleep electroencephalography. There was an overall increase in REM sleep duration during the administration of the microorganisms in the test groups. In particular, the test group 1 showed significant increase in REM sleep duration by 30 minutes on days 4 and 7 after the administration of the microorganisms as compared to the control group (p < 0.05). It is thought that this increase is due to a concomitant increase in light sleep duration in the test group 1, where total sleep duration itself increased the most.

FIG. 10 shows a result of comparing the change in sleep latency (recorded for 24 hours) as compared to the initially measured total sleep latency (time taken to fall asleep) by sleep electroencephalography. Although not significant, the overall decrease in sleep latency was greater in the test group 1 as compared to the control group. The animals of the test group 1 fell asleep about 25 minutes earlier. In addition, the administration of the microorganisms resulted in further decrease in sleep latency as compared to the control group on day 1 after administration of the microorganisms in the test groups 2 and 3, and on day 7 after the administration of the microorganisms in the test group 4.

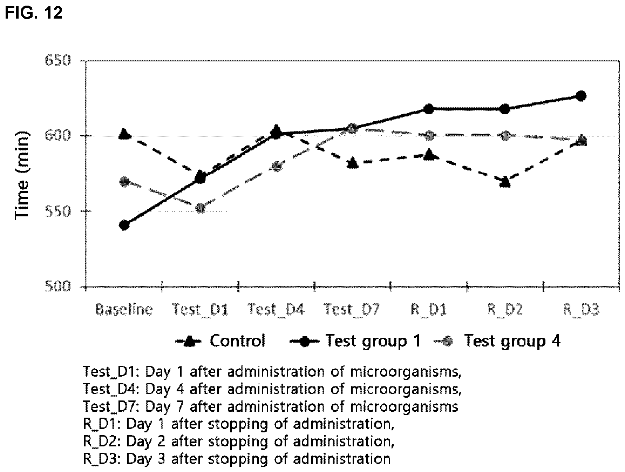

FIGS. 11 and 12 show the statistical analysis result of the change in sleep measurements as compared to the initial sleep measurements by sleep electroencephalography during and after the administration of the microorganisms for the control and test groups (including only the test group 1 and the test group 4).

FIG. 11 shows a result of investigating the change in sleep duration and wake time (recorded for 24 hours) of a control group, a test group 1 and a test group 4 by sleep electroencephalography. In the control group, total sleep duration decreased and increased from day 1 to day 7 after the administration of the microorganisms and after stopping of the administration of the microorganisms. But, in the end, the initial and last measured sleep duration were almost similar with no significant change. However, in the test group 1, sleep duration increased from day 1 after the administration of the microorganisms. It increased sharply on days 4 and 7, and the increase was maintained until day 3 after stopping of the administration. Although the magnitude of the increase was smaller than the test group 1, the test group 4 also showed increase in sleep duration on day 7 after the administration of the microorganisms. The increase was maintained until day 2 after stopping of the administration, and then decreased slightly on day 3. The control group did not show significant change in wake time. However, the test group 1 showed steady decrease in wake time by up to 111 minutes. The test group 4 also showed decrease by up to 42 minutes. It is thought that the decreased wake time resulted in sleep duration.

FIG. 12 shows a result of investigating the change in non-REM sleep duration (recorded for 24 hours) of different groups by sleep electroencephalography. In the control group, non-REM sleep duration decreased and increased from day 1 to day 7 after the administration of the microorganisms. But, in the end, the initially and last measured non-REM sleep duration were almost similar with no significant change. However, in the test group 1, sleep duration increased from day 1 after the administration of the microorganisms. It increased sharply on day 7, and the increase was maintained until day 3 after stopping of the administration. The test group 4 also showed increase in non-REM sleep duration until day 7 after the administration of the microorganisms. The level of non-REM sleep duration was maintained after stopping of the administration.

### 3. Result of animal experiment using extracellular vesicles (EVs)

Sleep EEG results were obtained for all the experimental albino rats. Initial sleep measurements were taken for a single day (test group 6) or averaged over two days (test group 5). Sleep measurements were recorded on days 1, 3 and 6 after EV administration. Although the results are limited in terms of statistical significance due to the small number of animals, the trend of change was evident.

FIG. 13 shows a result of investigating the change in sleep duration and wake time as measured by sleep electroencephalography (recorded for 24 hours). Total sleep duration increased from the first day of the EV administration and increased steadily until days 3 and 6. It was increased by approximately 25 minutes for the test group 5 and by about 43 minutes for the test group 6. On the other hand, wake time decreased steadily. The decreased wake time was translated into increased sleep duration. In the test group 6, although the EV dose was 2 mg, corresponding to about 1/2 of the test group 5, sleep duration increased 1.7 times greater.

FIG. 14 shows a result of investigating the change in non-REM sleep duration (recorded for 24 hours) as measured by sleep electroencephalography. Daytime sleep duration increased from the first day of EV administration and increased steadily until days 3 and 6. The non-REM sleep duration increased by approximately 30 minutes for the test group 5 and by about 40 minutes for the test group 6. The increase in sleep duration was entirely attributable to deep sleep as shown in FIG. 13.

Although the exemplary embodiments of the present disclosure have been described above, those skilled in the art can add, change or delete elements without departing from the spirit of the present disclosure as set forth in the patent claims. The present disclosure may be modified and changed in various ways, which will also be included within the scope of the present disclosure.

### [Accession number]

Depository institution: Korean Culture Center of Microorganisms
Accession number: KCCM13103P
Date of deposition: 20220111
Depository institution: Korean Culture Center of Microorganisms
Accession number: KCCM13104P
Date of deposition: 20220111
Depository institution: Korean Culture Center of Microorganisms
Accession number: KCCM13105P
Date of deposition: 20220111

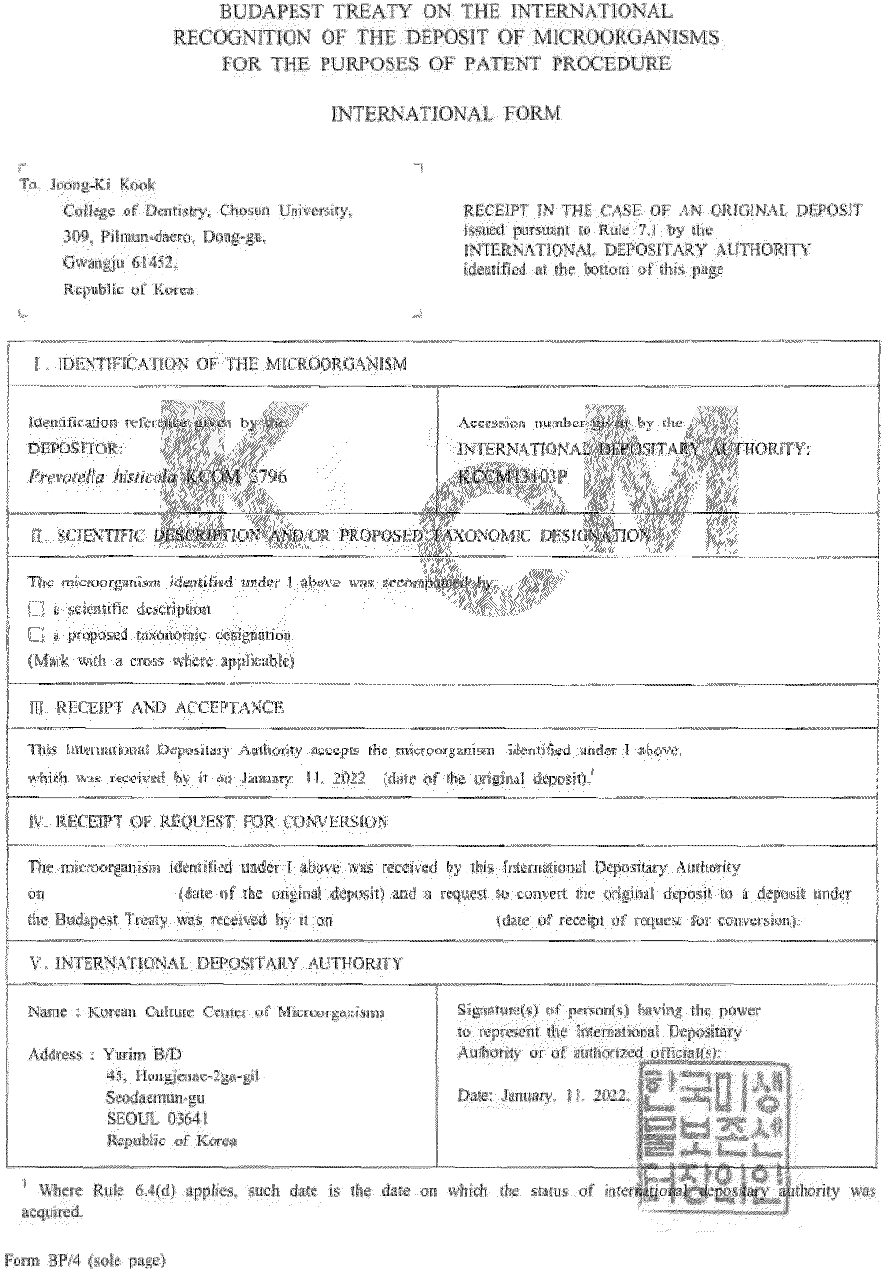
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Joong-Ki Kook
College of Dentistry, Chosun University,
309, Pilmun-daero, Dong-gu,
Gwangju 61452,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Prevotella histicola KCOM 3796 | Accession number given by the
INTERNATIONAL DEPOSITARY AUTHORITY:
KCCM13103P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on January. 11. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 11. 2022.

¹ Where Rule 6.4(d) applies, such date is the date on which the status of international depositary authority was
acquired.

Form BP/4 (sole page)

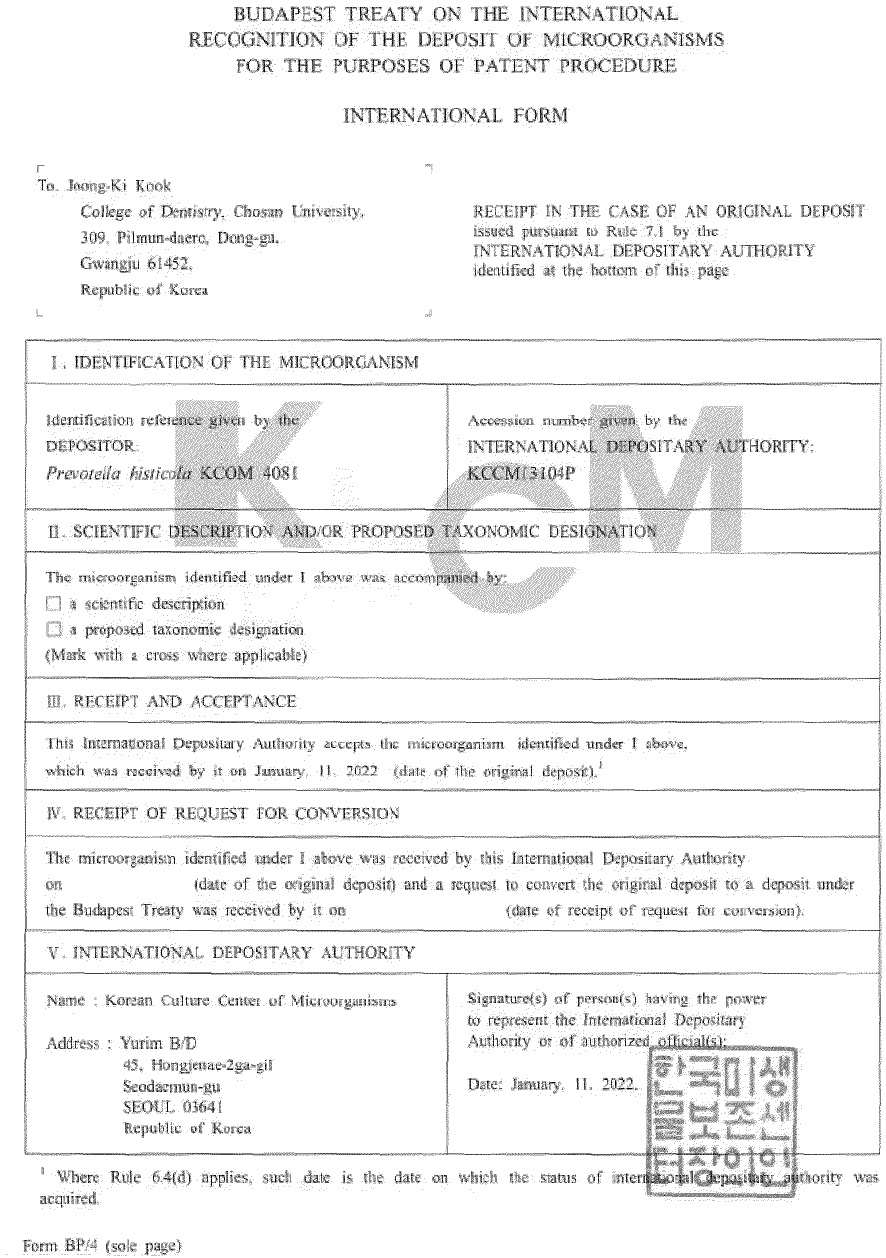
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Joong-Ki Kook
College of Dentistry, Chosun University,
309, Pilmun-daero, Dong-gu,
Gwangju 61452,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Prevotella histicola KCOM 4081 | Accession number given by the
INTERNATIONAL DEPOSITARY AUTHORITY:
KCCM13104P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on January. 11. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 11. 2022.

¹ Where Rule 6.4(d) applies, such date is the date on which the status of international depositary authority was
acquired.

Form BP/4 (sole page)

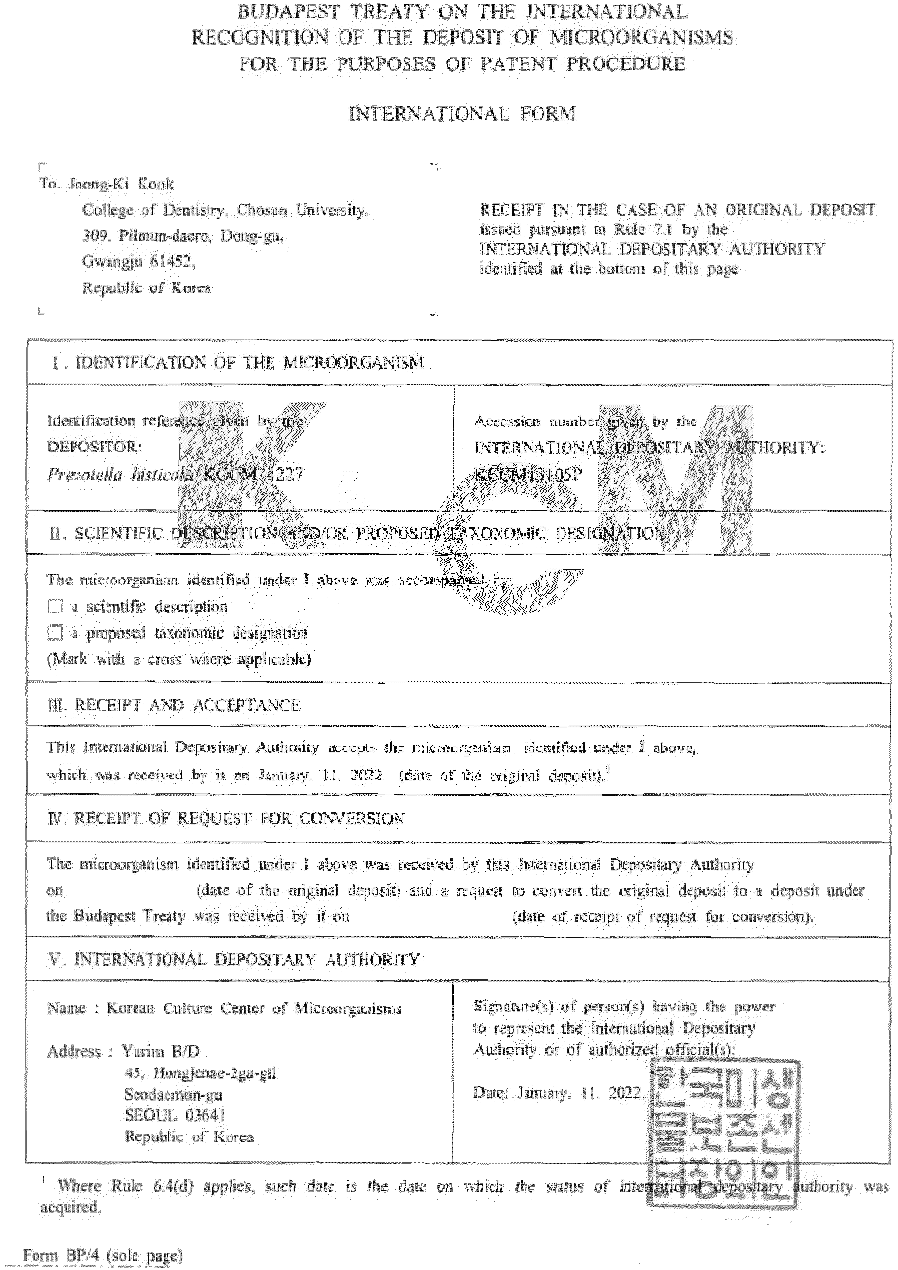
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Joong-Ki Kook
College of Dentistry, Chosun University,
309, Pilmun-daero, Dong-gu,
Gwangju 61452,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Prevotella histicola KCOM 4227 | Accession number given by the
INTERNATIONAL DEPOSITARY AUTHORITY:
KCCM13105P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on January. 11. 2022 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 11. 2022.

¹ Where Rule 6.4(d) applies, such date is the date on which the status of international depositary authority was
acquired.

Form BP/4 (sole page)

## Claims

1. *Prevotella histicola* exhibiting preventive, ameliorative or therapeutic activity in sleep disorder, wherein the *Prevotella histicola* is *Prevotella histicola* deposited with an accession number selected from a group consisting of accession numbers KCCM13103P, KCCM13104P and KCCM13105P.

2. The *Prevotella histicola* according to claim 1, wherein the *Prevotella histicola* is *Prevotella histicola* KCOM 3796 deposited with an accession number KCCM13103P.

3. The *Prevotella histicola* according to claim 1, wherein the *Prevotella histicola* is *Prevotella histicola* KCOM 4081 deposited with an accession number KCCM13104P.

4. The *Prevotella histicola* according to claim 1, wherein the *Prevotella histicola* is *Prevotella histicola* KCOM 4227 deposited with an accession number KCCM13105P.

5. The *Prevotella histicola* according to claim 1, wherein the *Prevotella histicola* is isolated from oral saliva.

6. The *Prevotella histicola* according to claim 1, wherein the 16S rDNA sequence of the *Prevotella histicola* comprises a sequence selected from a group consisting of SEQ ID NOS 3, 4 and 5.

7. The *Prevotella histicola* according to claim 1, wherein the sleep disorder is one or more selected from a group consisting of insomnia, breathing-related sleep disorder, and circadian rhythm sleep disorder.

8. The *Prevotella histicola* according to claim 7, wherein the insomnia is one or more selected from a group consisting of sleep onset insomnia, sleep maintenance insomnia and sleep terminal insomnia.

9. The *Prevotella histicola* according to claim 7, wherein the breathing-related sleep disorder is one or more selected from a group consisting of obstructive sleep apnea syndrome, central sleep apnea syndrome, and central alveolar hypoventilation syndrome.

10. The *Prevotella histicola* according to claim 7, wherein the circadian rhythm sleep disorder is one or more selected from a group consisting of a delayed sleep phase type, an advanced sleep phase type, a jet lag type, and a shift work type.

11. The *Prevotella histicola* according to claim 1, wherein the prevention, amelioration or treatment of sleep disorder is achieved by increasing total sleep duration.

12. The *Prevotella histicola* according to claim 1, wherein the prevention, amelioration or treatment of sleep disorder is achieved by increasing non-REM (NREM) sleep duration.

13. The *Prevotella histicola* according to claim 1, wherein the prevention, amelioration or treatment of sleep disorder is achieved by increasing REM sleep duration.

14. The *Prevotella histicola* according to claim 1, wherein the prevention, amelioration or treatment of sleep disorder is achieved by reducing sleep latency.

15. The *Prevotella histicola* according to claim 1, wherein the prevention, amelioration or treatment of sleep disorder is achieved by reducing total wake time.
